# EUROPÄISCHE PATENTSCHRIFT

(11) **EP 1 052 897 B1**
(45) Veröffentlichungstag und Bekanntmachung des Hinweises auf die Patenterteilung: **10.09.2008**
(21) Anmeldenummer: 98906879.6
(22) Anmeldetag: 16.01.1998
(51) Int. Cl.: A01K 67/027, C12N 5/06, C12N 5/10

(54) **EFFICIENTER KERNTRANSFER MIT PRIMORDIALEN KEIMZELLEN**
EFFICIENT NUCLEAR TRANSFER USING PRIMORDIAL GERM CELLS
TRANSFERT DE NOYAU EFFICACE AVEC DES CELLULES GERMINALES PRIMORDIALES

(43) Veröffentlichungstag der Anmeldung: 22.11.2000
(73) Patentinhaber: Agrobiogen GmbH, Larezhausen, 86567 Hilgertshausen (DE)
(72) Erfinder: Brem, Gottfried, 86567 Hilgertshausen (DE); Durcova-Hills, Gabriela, 85764 Oberschleissheim (DE); Müller, Sigrid, Inst. Tierzucht und Genetik, AT, 1210 Wien (AT); Schernthaner, Wolfgang, 85764 Oberschleissheim (DE); Wenigerkind, Hendrik, Inst. Tierzucht Genetik,AT, 1210 Wien (AT); Wolf, Eckhard, Inst. Tierzucht und Genetik, AT, 1210 Wien A-1210 Wien (AT); Zakhartchenko, Valeri, 85764 Oberschleissheim (DE)
(74) Vertreter: Becker Kurig Straus
(86) Internationale Anmeldenummer: PCT/EP1998/000229
(87) Internationale Veröffentlichungsnummer: WO 1999/035906

(56) Entgegenhaltungen:
- EP-A- 0 451 823
- EP-A- 0 774 510
- WO-A-95/10599
- WO-A-97/07668
- WO-A-97/25412
- DELHAISE F ET AL.: "Nuclear transplantation using bovine primordial germ cells from male fetuses" REPRODUCTION FERTILITY AND DEVELOPMENT, Bd. 7, Nr. 5, 1995, Seiten 1217-1219, XP002079413 EAST MELBOURNE AU
- BREM G ET AL: "MAMMARY GLAND SPECIFIC EXPRESSION OF CHYMOSIN CONSTRUCTS IN TRANSGENIC RABBITS" THERIOGENOLOGY, Bd. 43, Nr. 1, Januar 1995, Seite 175 XP002070795

## Beschreibung

Die vorliegende Erfindung betrifft ein Verfahren zum Züchten von Tieren über Klonierung. Diese Erfindung betrifft insbesondere ein Verfahren zum Klonieren von Tieren über einen effizienten Kerntransfer mit bestimmten fetalen Zellen.

Tiere, insbesondere Nutztiere, werden vom Menschen seit langem für die verschiedensten Zwecke weitergezüchtet und im Hinblick auf bestimmte Eigenschaften fortentwickelt. So wurden beispielsweise Kühe und Stiere mit hohem Zuchtwert für Milchleistung weiterverpaart, um Tiere mit hohem Milchertrag zu erhalten.

In den letzten Jahren gerieten Tiere, insbesondere Vertreter der Ungulaten, wie Schafe, Rinder bzw. Kühe auch als Produktionsstätten ernährungsphysiologisch bzw. pharmazeutisch bedeutender Stoffe in den Mittelpunkt des Forschungsinteresses, da es mit der Entwicklung der Gentechnologie möglich wurde gezielt Tiere herzustellen, denen eine neue Eigenschaft, beispielsweise die Fähigkeit zur Produktion eines bestimmten Arzneimittels, verliehen werden konnte. Das Problem bei der wirtschaftlichen Nutzung derartiger Tiere besteht jedoch darin, daß das ihnen transferierte Genkonstrukt integriert und stabil an die Nachkommen weitergegeben wird.

Zu diesem Zweck wurde versucht, das Problem des Gentransfers dadurch zu lösen, daß dieser in Zellen vorgenommen wird, wobei aus diesen Zellen mittels Klonierung wieder Tiere generiert werden.

In der Fachwelt wird der Begriff des "Klonierens" allgemeinen als Vervielfältigung eines genetischen Materials, abgeleitet von einer einzigen Zelle definiert, was übertragen auf die Embryologie als Erstellung von Embryonen bzw. Tieren mit identischem Genotyp verstanden werden kann. In der Embryologie wird der Keimling während der Blastogenese, also bis zur Entwicklung der Anlage von Primitivorganen als Embryo, in den nachfolgenden Entwicklungsstufen als Fetus bezeichnet. Embryonale Phasen verlaufen je nach Spezies in unterschiedlich langen Zeiträumen ab, so beispielsweise beim Rind in einem Zeitraum von etwa 4 Wochen, wobei bei anderen Spezies innerhalb der Ungulaten kürzere oder auch längere Zeiträume dafür erforderlich sein können.

Zur Klonierung von Tieren, also zur Vervielfältigung eines einem bestimmten Tier eigenen Genotyps wurden bis dato mehrere Wege beschritten.

Einerseits wurden frühe Embryonalstadien und Fortentwicklungen einem mikrochirurgischen Eingriff unterworfen und die jeweils daraus isolierten Teile wurden in vitro bzw. in vivo weitergezüchtet.

Weiter wurde eine als "Chimäres Klonen" bezeichnete mikromanipulatorische Kombination asynchroner Entwicklungsstadien durchgeführt, bei der Blastomere(n) aus Embryonen weiter fortgeschrittener Stadien mit Blastomeren aus früheren Stadien zusammengebracht wurden, mit dem Ziel, die erstgenannten in ihrer Weiterentwicklungskapazität zu unterstützen und somit identische Viellinge zu erzeugen. Die größte Anzahl damit erhaltener Klone lag jedoch lediglich in einer Größenordnung von maximal 5-8.

Eine weitere Vorgehensweise war die parthenogenetische Aktivierung oder die Verpaarung homozygoter Elterntiere, um hinsichtlich bestimmter Eigenschaften Klone zu erhalten.

Da sich die oben genannten Verfahren hinsichtlich Ihrer Effektivität und Zuverlässigkeit jedoch als relativ schlecht erwiesen, wurde ein weiteres Verfahren entwickelt, das generell als Kerntransfer bezeichnet wird.

Dabei werden Zellkerne, die von mehrzelligen Embryonen stammen, in entsprechend vorbereitete Eizellen überführt, wobei genetisch identische Embryonen erstellt werden konnten.

Um eine Klonierung mittels Kerntransfer erfolgreich durchführen zu können, müssen jedoch einige unabdingbare Parameter berücksichtigt werden.

Die Eizelle, die als Empfängerzelle eingesetzt wird, muß das Metaphase-Stadium in der 2. Reifeteilung (Metaphase II) vollendet haben und soll keine eigene nukleare DNA mehr enthalten, d.h. sie soll als sogenannte enukleierte Eizelle vorliegen. Des weiteren sollte das Cytoplasma der Eizelle so wenig wie möglich beeinflußt werden, da die in dem Cytoplasma selbst enthaltenen Stoffe für die Weiterentwicklung, beispielsweise die Teilung der Zelle, von Bedeutung sein können.

Weiterhin muß die nukleare DNA des übertragenen Kerns reprogrammiert werden. Da der (Spender-) Kern von einem mehrzelligen Embryo stammt, hat die jeweilige Spenderzelle bereits einige Teilungszyklen durchlaufen. Dies bedeutet, daß sich die Zelle in einem gegenüber einer totipotenten befruchteten Eizelle fortgeschrittenen Entwicklungsstadium befindet, in dem möglicherweise bereits bestimmte, für die frühe Entwicklung erforderliche Gene abgeschaltet sind.

Aus diesem Grund muß die verwendete Kern-DNA derart reprogrammiert werden, daß die vollständige genetische Information der Kern-DNA wieder zur Verfügung steht und das Teilungsschema des Embryos wieder beim Stadium der Zygote beginnt. Je besser somit diese Reprogrammierung bzw. Aktivierung erreicht werden kann, desto höher liegt die Wahrscheinlichkeit einer erfolgreichen Klonierung, mit der dann auch ein fertig entwickeltes, d.h. lebend geborenes geklontes Tier erhalten werden kann.

Neben der Kern-DNA ist u.a. auch die in dem Cytoplasma vorhandene mRNA von Bedeutung, da diese im Zeitpunkt der Vereinigung von Eizelle und Spenderzelle die für das gegenwärtige Entwicklungs- bzw. Differenzierungsstadium der Spenderzelle erforderlichen Botschaften darstellt und die damit hergestellten Proteine einen Einfluß auf die weitere Entwicklung der Zelle nehmen können.

Das Verfahren des Kerntransfers wurde bereits mit bescheidenem Erfolg eingesetzt. So berichteten Willadsen et al. (Nature 320 (1986), 63-65) über die Klonierung von Lämmern, wobei die Kerne aus Kern-Spenderzellen aus dem 8-Zell-Stadium stammten. Robl et al. (J. Anim. Sci. 64 (1987), 642-647) berichteten über die ersten Kerntransferexperimente beim Rind, wobei ausschließlich mit ex vivo gewonnenen Rinderembryonen als Kernspender gearbeitet wurde. Bei diesen Versuchen war immer eine in vivo Zwischenkultur in Schafeileitern erforderlich. In den folgenden Jahren konnte auch gezeigt werden, daß das Embryonalklonen beim Rind rein in vitro, also unter Verwendung von in vitro produzierten Embryonen und in vitro gereiften Eizellen, erfolgreich durchgeführt werden kann (Sims et al., Proc. Natl. Acad. Sci. USA 91 (1991), 6143-6147).

In der WO 97/07668 wird weiter ein Verfahren zur Wiederherstellung eines tierischen Embryos beschrieben, bei dem generell ein Kern mit einem diploiden Chromosomensatz in eine enukleierte Eizelle überführt wird, die in dem Metaphasenstadium II gehalten wird, wobei die Eizelle beim Einbringen des Kerns erst nach einer gewissen Zeit aktiviert wird. Durch später erfolgende Aktivierung der Eizelle nach Einbringen der Kern-DNA soll eine verbesserte Reprogrammierung der eingebrachten Kern-DNA erreicht werden.

Die WO 96/07732 beschreibt ebenfalls ein Verfahren zur Wiederherstellung eines tierischen Embryos, bei dem Zellen aus der Keimscheibe eines Embryos im Blastozysten-Stadium isoliert, in einer geeigneten Umgebung reifen gelassen und deren Kerne dann in geeignete Zellen eingebracht werden.

Ein Problem bei dieser Technologie besteht jedoch immer noch darin, geeignete SpenderZellen für den Kerntransfer zu finden, mit der sich ein tierischer Embryo am zweckmäßigsten und wirtschaftlichsten herstellen läßt. Bekanntermaßen stellt die Reprogrammierung der Kern-DNA aus der jeweils gewählten Spenderzelle die größte Schwierigkeit bei der Embryoklonierung dar, da diese nicht nur Einfluß hat auf die weitere frühe Reifung des Embryos, sondern auch auf die spätere Entwicklung nach einer gegebenenfalls durchgeführten Einpflanzung in ein Muttertier. So bestehen trotz aller Erfolge auf diesem Gebiet immer noch Probleme hinsichtlich einer effektiven Reprogrammierung der Spender-Kern-DNA, um die manipulierte Eizelle mit neuem Kern dem Zustand einer natürlichen Zygote anzunähern. Dies drückt sich u.a. in einer äußerst geringen Ausbeute hinsichtlich der Gewinnung embryonaler Blastozysten und einer geringen Teilungsrate aus.

Eine Aufgabe der vorliegenden Erfindung besteht daher darin, die Nachteile des Standes der Technik zu überwinden und eine geeignete Kern-Spenderzelle zur Verfügung zu stellen, mit der ein verbessertes Verfahren zum Klonieren von Tieren bereitgestellt werden kann.

Diese Aufgabe wird gelöst durch ein Verfahren zum Klonieren eines tierischen Embryos, bei dem als Spenderzelle für den Kerntransfer primordiale Keimzellen eingesetzt werden. Der Kern dieser Zelle wird mit einer geeigneten Empfängerzelle zusammengebracht und die so erhaltene Zelle wird für einen Zeitraum gezüchtet, damit sich eine Blastozyste bildet. Die daraus erhältliche Blastozyste kann dann gegebenenfalls zum Austragen in ein Muttertier eingebracht werden.

Gemäß einer bevorzugten Ausführungsform der vorliegenden Erfindung ist die Empfängerzelle eine enukleierte Eizelle, in die der Kern der primordialen Keimzelle zum Kerntransfer eingebracht werden kann oder mit der die primordiale Keimzelle selbst fusioniert wird. Die primordiale Keimzelle kann dabei aus einem Fetus und direkt oder nach längerem Züchten verwendet werden.

Die Tiere, bei denen das erfindungsgemäße Verfahren durchgeführt werden kann, sind beispielsweise Ungulaten, Kaninchen, Nager, oder Vögel, wobei Ungulaten, insbesondere Schweine, Schafe, Ziegen, Rinder bzw. Kühe bevorzugt sind.

In einer bevorzugten Ausführungsform sind die in dem Verfahren eingesetzten primordialen Keimzellen transgen, d.h. sie enthalten ein oder mehrere Gene, die entweder von einer exogenen Quelle abgeleitet sind oder die ein endogenes, an einen anderen, nicht natürlichen Locus im Genom eingebrachtes Gen darstellen. Diese Gene codieren vorzugsweise für ein Arzneimittel, beispielsweise einen Antikörper oder einen ernährungsphysiologisch interessanten Stoff, beispielsweise Chymosin oder Trypsin, wobei die Gene jeweils unter der Kontrolle eines oder des endogenen oder eines exogenen Promotors liegen können.

Zur Gewinnung primordialer Keimzellen werden Feten aus graviden Tieren gewonnen, beispielsweise durch Absaugen aus dem Fetus. Die aus dem Fetus gewonnen Zellen werden sodann auf die gewünschten primordialen Keimzellen selektiert, wie beispielsweise durch Anhaftenlassen von vorhandenen Fibroblasten an das Kulturgefäß, Wachsenlassen der Keimzellen auf geeigneten Feederzellen oder mechanische Selektion mittels einer Pipette. Primordiale Keimzellen lassen sich aufgrund ihres Phänotyps von anderen Zellen unterscheiden, da sie sich als leicht gelblich aussehende, irregulär oder rund geformte Zellen mit großem Kern identifizieren lassen, die gegebenenfalls "Blebbing"-Phänomene zeigen. Damit können dann die gewünschten primordialen Zellen isoliert werden.

Für die nachfolgenden Schritte kann die gewonnene primordiale Keimzelle als solche eingesetzt werden, oder der Kern kann daraus isoliert und weiterverwendet werden.

Als Empfängerzellen werden in der Regel enukleierte Eizellen eingesetzt, die in vivo oder in vitro gereift sind. So können beispielsweise in vitro gereifte, unbefruchtete Eizellen eingesetzt werden, bei denen nach Erreichen der Metaphase II die umgebenden Cumuluszellen entfernt wurden.

Die Empfängerzelle soll vorzugsweise keine eigene Kern-DNA aufweisen. Für die Entfernung der Eizell-DNA sind im Stand der Technik mehrere Möglichkeiten vorhanden, wie beispielsweise die Trennung der Eizelle in zwei Hälften, von denen eine Hälfte keinen Kern mehr aufweist und weiterverwendet werden kann, oder eine Bestrahlung mit ultraviolettem Licht zur Zerstörung der zelleigenen DNA. Eine Entfernung des Kerns bzw. der Pro-Nuclei oder der Metaphasen-Platte mittels Mikromanipulation ist ebenfalls möglich. Als bevorzugt hat sich eine Behandlung der Eizellen vor der Mikromanipulation mit Cytochalasin B mit anschließendem Absaugen des in der Nähe des Polkörpers liegenden Zytoplasmas mit Hilfe einer Pipette, beispielsweise mit einem Leitz-Micromanipulator (Leica, Bensheim, Deutschland) geführt, erwiesen. Da die Kern-DNA der Eizelle zu diesem Zeitpunkt in der Nähe der Polkörperchen lokalisiert ist, ist die Enukleationsrate bei dieser Methode sehr hoch, wobei gleichzeitig nur ein kleiner Teil des Cytoplasmas mit abgesaugt wird.

Nach Gewinnen der jeweils beim Kerntransfer beteiligten Zellen können generell zwei Wege beschritten werden. Der Kern der primordialen Keimzellen wird anhand im Stand der Technik bekannter und etablierter Verfahren isoliert und in die vorbereitete Empfängerzelle eingebracht, wie beispielsweise mittels Injektion oder die primordiale Keimzelle selbst wird mit der Empfängerzelle fusioniert.

Bei einer Fusion kann eine primordiale Keimzelle mit Hilfe einer geeigneten Vorrichtung, wie einer Transferpipette unter die Zona pellucida der enukleierten Eizelle geschoben und dort abgesetzt werden. Zur Integration des Zellkerns der primordialen Keimzelle in das Zellplasma der Eizelle wird die Membran der Fibroblast mit der Membran der Eizelle fusioniert. Techniken zur Fusion von Zellen sind im Stand der Technik wohlbekannt, beispielsweise Fusion unter Verwendung des Sendai-Viruses, Behandlung mit PEG (Polyethylenglycol), Laserfusion oder Elektroschock. Die letztgenannte Methode, die sogenannte Elektrofusion, bei der durch gegebenenfalls mehrmalige, beispielsweise 2 bis 10 mal, kurzzeitige Gleichstrompulse von etwa 1 bis 5 kV/cm, vorzugsweise 1 bis 3 kV/cm, mit einer jeweiligen Dauer von 2 µsek. bis 1 sek., Poren induziert werden, die ein Zusammenfließen des Zytoplasmas ermöglichen, ist in dem vorliegenden Verfahren bevorzugt, da die elektrischen Pulse bei geeigneter Stärke gleichzeitig eine Aktivierung der (fusionierten) Eizelle mit sich bringen können. Die Aktivierung kann auch einige Stunden (ca. 2-5 Std.) nach der Fusion erfolgen, beispielsweise durch eine Inkubation der fusionierten Zelle in einer 7 %igen Alkohollösung, vorzugsweise einer 7 %igen Ethanollösung, oder anhand anderer im Stand der Technik bekannten Verfahren.

Die Aktivierung der fusionierten Zelle ist ein wichtiger Schritt, da sie die Voraussetzung für das Ingangkommen der Teilungsaktivität des Fusionsproduktes ist. Nach erfolgter Fusion und Aktivierung werden die Keimzellen-Eizelle-Komplexe (Kerntransferembryonen) gezüchtet, bis sie ein Stadium erreichen, in dem sie gegebenenfalls auf einen Empfänger transferiert werden können. Dabei können dem verwendeten Kulturmedium nach Wahl Stoffe zugesetzt werden, die die Aggregation von Mikrotubuli unterstützen oder inhibieren. Nocadazol sowie Colcemid sind Beispiele für die Aggregation inhibierende Mittel, Taxol ist ein Mikrotubuli-Stabilisator. Diese Stoffe verhindern eine gegebenenfalls auftretende Bildung mehrerer Pro-Nuclei.

Bei den bestehenden Verfahren des Standes der Technik mußten zur weiteren Züchtung der sich bildenden Embryos diese sorgfältig in einen Zwischenempfänger überführt werden.

Dies wurde im allgemeinen dadurch erreicht, daß der Embryo in einem schützenden Medium, wie Agar, verpackt in die Eileiter eines "einstweiligen Muttertiers" (temporärer Empfänger) überführt wurde, in dem eine weitere Entwicklung bis zur Einpflanzung in das (endgültige) Muttertier erforderlich war. Bei dem erfindungsgemäßen Verfahren ist es jedoch auch möglich, bestehende in-vitro-Systeme für die Kultivierung einzusetzen, ohne dabei die Ausbeuten zu verschlechtern. Ohne an eine Theorie gebunden zu sein, könnte diese Tatsache mit der getroffenen Auswahl der Spenderzelle einhergehen, mit der Embryonen erhalten werden können, die hinsichtlich Ihrer Entwicklung natürlich erzeugten Embryonen sehr nahe kommen. Die Zellen werden für einen Zeitraum in Kultur gehalten, bis sich Blastozysten bilden. Dies umfaßt einen Zeitraum von bis zu 10 Tagen, vorzugsweise 6 bis 7 Tagen.

Erfindungsgemäß ist es nun möglich klonierte Embryonen zu Feten heranwachsen zu lassen, die dann wieder als Kernspender herangezogen werden können. Im Rahmen dieser sogenannten Reklonierung kann die Zahl an klonierten Embryonen weiter erhöht werden.

Die primordialen Keimzellen zum Einsatz in dem erfindungsgemäßen Verfahren können aus einer Vielzahl von Tieren gewonnen werden, wie beispielsweise aus Säugern, Ungulaten, Kaninchen, Nagern, wie beispielsweise Ratten oder Mäusen, oder Vögeln, wie beispielsweise Enten, Gänsen oder Hühnern. Dabei sind u.a. im Hinblick auf wirtschaftliche Gesichtspunkte Ungulaten bevorzugt, wie beispielsweise Rinder, Schafe, Ziegen, Büffel, Kamele sowie Schweine. Am meisten bevorzugt sind Schafe bzw. Kühe.

Um die Isolierung des Genprodukts zu erleichtern kann das Genprodukt in ein Produkt des Tieres selbst gerichtet werden, bei Kühen bzw. Schafen beispielsweise in die Milch oder bei Vögeln in die Eier. Dies kann durch Wahl geeigneter Promotoren zur organspezifischen Expression erreicht werden, die im Stand der Technik bekannt sind. Das Genprodukt kann jedoch gleichermaßen aus dem Tier selbst gewonnen werden, beispielsweise aus dem Serum. Auch ist es möglich, daß das/die Organ(e)/Gewebe der Tiere das gewünschte Produkt darstellen, beispielsweise für eine (Xeno-)Transplantation.

Die in dem erfindungsgemäßen Verfahren eingesetzten primordialen Keimzellen bzw. die Feten oder Tiere, von denen sie abgeleitet sind, können darüber hinaus transgen sein, wobei das Transgen vorzugsweise für ein ernährungsphysiologisch oder pharmazeutisch interessantes Produkt, beispielsweise einen Antikörper codiert. Beispielsweise können bei Kühen oder Schafen die Gene für Chymosin oder Trypsin in ein Konstrukt eingebaut werden, das die Produktion des entsprechenden Enzyms, bzw. eines seiner Vorläufer in der Milch des Tieres ermöglicht. Das Transgen von Interesse kann dabei, je nach Wunsch, unter der Steuerung eines exogenen, ebenfalls transgenen Promotors liegen, oder ein bekannter endogener Promotor kann für diesen Zweck eingesetzt werden.

Mit dem erfindungsgemäßen Verfahren ist es nun möglich, eine Verbesserung der Gewinnung homologer tierischer Proteine, eine Veränderung tierischer Produkte, wie Milch selbst oder die Gewinnung tierischer Organe für beispielsweise einen medizinischen Einsatz zu erreichen.

Eine ganze Reihe von Proteinen wird bislang aus tierischen Organen durch Aufreinigung aus diesen Organen gewonnen und dann in der Medizin oder Technik eingesetzt. Probleme dabei bestehen u.a. hinsichtlich der relativen Mengen, in denen sie in diesen Geweben vorhanden sind (beispielsweise FSH aus Hypophysen), was zu hohen Produktionskosten führt, da zur Gewinnung einerseits eine große Menge an Ausgangsmaterial, d.h. viele Tiere, erforderlich sind, was aufgrund der Vielzahl von Tieren die Gefahr einer Kontamination, mit beispielsweise Krankheitserregern, wie BSE oder Ehec, mit sich bringt.

Beispiele für interessierende Proteine aus Tierorganen sind Aprotinin aus der Lunge, Chymsin aus dem Magen, Katalase aus der Leber, Elastase, Pankreatin, Insulin oder Trypsin aus dem Pankreas, Hyaluronidase aus Hoden, Chondroitin aus Trachea, Kollagene aus der Haut, Fibronectin oder Vitronectin aus Plasma, Epithelial Cell Growth suppl. oder LH (Luteinisierendes Hormon) aus der Hypophyse, Fibroblast growth factor oder Ganglioside aus dem Hirn, sowie Hämoglobin, Thrombin, Transferrin usw.. Diese Aufzählung ist nicht als Einschränkung anzusehen.

Für alle diese Produkte kann eine ektotope Expression, d.h. eine Expression in einem anderen Gewebe, beispielsweise in der Milchdrüse erreicht werden, wenn in den für die Klonierung verwendeten Zellen vorher entweder ein additiver Gentransfer durchgeführt wurde, beispielsweise über Injektion, Transformation, Transfektion oder anhand eines anderen im Stand der Technik bekannten Verfahrens, wobei ein in vitro rekombiniertes Genkonstrukt zusätzlich ins Genom integriert wird. Darüber hinaus kann durch homologe Rekombination in den Zellen erreicht werden, daß das endogen vorhandene Gen mit einem Promotor gekoppelt wird, der für dieses Strukturgen ein anderes Expressionsmuster ergibt, beispielsweise Produktion von Chymosin im Euter mit einhergehender Sezernierung in die Milch anstelle der endogenen Synthese im Magen. Weiter kann ein endogen vorhandener Promotor, beispielsweise der Casein- oder Lactoglobulin-Promotor mit einem neuen Strukturgen gekoppelt werden, so daß für die Expression optimale Bedingungen vorliegen. In beiden vorstehend aufgeführten Fällen können Promotor und Strukturgen, die homolog in das Genom hinein rekombiniert werden, vorab aus einer Genbank isoliert werden, die beispielsweise aus primordialen Keimzellen gewonnen worden ist, so daß nicht nur spezieseigene DNA (Selbstklonierung) eingesetzt wird, sondern es sich auch um isogene DNA handelt.

Auf diesem Weg kann daher die Zusammensetzung von Nahrungsmitteln, die aus tierischen Produkten, wie beispielsweise Milch gewonnen werden, in gewünschter Weise verändert werden, so daß diese positive alimentäre, dietätische, gesundheitsfördernde Eigenschaften oder ein geringeres allergenes Potential, bessere Lagerungsstabilität oder Verarbeitungseigenschaften aufweisen. So kann beispielsweise Milch mit Ehec-Antikörpern oder Milch mit speziell auf Erkrankungen, wie beispielsweise Laktoseintoleranz, abgestimmten Eigenschaften hergestellt werden.

Darüber hinaus können durch Verwendung von MACs (Mammalian artificial Chromosomes) Integrationsmutationen vermieden und große DNA-Fragmente transferiert werden. Diese MACs werden als zusätzliche Mini- bzw. Mikrochromosomen im Kern genauso repliziert, wie die endogenen Chromosomen. Dadurch ist es beispielsweise möglich, über die Spezies hinweg Gencluster zu transferieren, beispielsweise komplette Immunglobulin-Gencluster des Menschen auf Nutztiere, wobei dieses Nutztier dann in der Lage wäre, humane Antikörper zu produzieren, die gewonnen und genutzt werden könnten. Der Transfer bestimmter MACs aus der eigenen Spezies führt weiter dazu, daß bei additiven Geneffekten eine Erhöhung der Synthese des Genprodukts folgern würde.

Wichtig ist auch eine Expression homologer Proteine bzw. auch von ggf. transgenen Geweben oder Organen in Nutztieren, bei Proteinen in den gleichen Organen, in denen diese Proteine auch beim Menschen exprimiert werden. Die Proteine werden dann anhand im Stand der Technik bekannter Verfahren gewonnen, die Gewebe bzw. Organe vor einer eventuellen Transplantation aus dem Tier entnommen. Der Vorteil dieser Vorgehensweise besteht in einer hohen Identität der exprimierten Proteine, da sie im richtigen Organ prozessiert bzw. post-translational modifiziert werden, beispielsweise Expression von Erythropoietin in der Niere. Die führt dazu, daß die aus den unterschiedlichen Geweben gewonnenen Proteine die gleiche Glycosylierung aufweisen, wie die Stoffe im Menschen selbst, wobei deren Aktivität der des natürlichen Proteins sehr nahe kommt. So können transgene Tiere, beispielsweise Schweine, Rinder usw. erhalten werden, die menschliches Insulin, Erythropoietin usw. produzieren, die in der Medizin dann besser genutzt werden können.

Anhand des erfindungsgemäßen Verfahrens kann ein einmal transgen gemachtes Nutztier mit beispielsweise den vorstehend aufgeführten Eigenschaften stabil fortgepflanzt werden.

So kann eine effiziente Gewinnung größerer Klongruppen transgener Tiere durch nachstehenden Verfahrensablauf wie folgt schematisch dargestellt werden:

### 1. Mikromanipulation von Zygoten

Dabei wird ein vorbereitetes Genkonstrukt (beispielsweise wie in der DE-OS-40 12 526 beschrieben) in eine befruchtete Eizelle eingeführt und integriert sich in das Genom. Dies stellt einen normalen Gentransfer mit einer additiven, zufälligen Integration des Genkonstrukts in das Genom der Zygote dar. Ebenfalls möglich ist die Durchführung einer homologen Rekombination, bei der gleichzeitig ein endogenes Gen ausgeschaltet oder ein endogener Promotor/ein endogenes Gen genutzt werden kann.

### 2. in vitro Kultur

Die Zygote wird kurzfristig, d.h für 1 - 5 Std. oder für mehrere Tage, beispielsweise 1 bis 8 Tage, in einem geeigneten Medium gezüchtet. Sie kann auch sofort in den Eileiter eines geeigneten Empfängertiers transferiert werden.

### 3. Transfer auf Empfänger

Die Zygote wird zur weiteren Entwicklung zu einem Embryo in den Eileiter oder den Uterus eines Empfängertier transferiert, wobei bis zu 4 Embryonen transferiert werden können

### 4. Gewinnung des Embryos bzw. Fetus

Der Fetus wird isoliert, beispielsweise durch Schlachtung des Empfängertiers und Entnahme des Fetus. Andere Arten der Gewinnung des Embryos ohne Tötung des Empfängertiers sind ebenfalls möglich.

### 5. Selektion auf transgene Föten

Zellen des Embryos werden gewonnen und auf Integration des Genkonstrukts untersucht.

### 6. Isolierung primordialer Keimzellen aus den Föten

Die primordialen Keimzellen aus Feten, die das Genkonstrukt integriert haben, werden gewonnen und weiterverwendet.

### 7. Klonierung

Die Kerne transgener, primordialer Keimzellen werden mit geeigneten Empfängerzellen zusammengebracht (Kerntransfer, Fusionierung) und wie hier näher erläutert behandelt.

### 8. in vitro Kultur

Die Zellen werden für einen Zeitraum von etwa 4 bis 10 Tagen in vitro gezüchtet.

### 9. Transfer auf Empfänger

Die sich bildenden Zellhaufen (Blastozysten) werden erneut auf Empfängertiere übertragen. Vor dem Transfer wird die Zona pelucida aufgeschlitzt, was sich als besonders geeignet erwiesen hat.

### 10. Wiederholen des Verfahrens von 6. bis 9.

Ein Vorteil des erfindungsgemäßen Verfahrens ist neben einer gesteigerten Effizienz, d.h. einer gegenüber den Verfahren des Standes der Technik gesteigerten Erfolgsrate bei der Wiederherstellung genetisch gleicher Embryonen auch darin zu sehen, daß größere Klongruppen erhältlich sind. So kann ein bereits geklonter Fetus dazu verwendet werden weitere Klone zu erzeugen, da die fetalen Zellen wiedergewonnen und in vitro effizient weiterverwendet werden können (Punkt 10 des vorstehend aufgeführten Verfahrens).

So konnten unter Verwendung des erfindungsgemäßen Verfahrens Ergebnisse erzielt werden, die sogar besser waren als jene, die aus punktierten Oozyten mit anschließender Reifung und Fertilisation - jedoch ohne Klonierung - entstanden sind. Die dabei beobachtete höhere in vivo Entwicklungskapazität erhöht die Effizienz der Klonierungsprogramme erheblich.

Als Maß für die Effizienz derartiger Verfahren kann die sogenannte Graviditätsrate (Trächtigkeitsrate) dienen, die als Anteil der nach Transfer von 6 - 7 Tagen in vitro kultivierten Embryonen auf synchronisierte Empfängertiere gravid gewordenen Tiere ermittelt werden kann. Die jeweiligen Graviditätsraten können durch Messung des Progesteronspiegels, Ultraschalluntersuchungen oder mittels rektaler Palpation bestimmt werden.

Dabei wurden für das Beispiel Rind mit unterschiedlichen Verfahren folgende Ergebnisse erhalten:

### Graviditätsraten:

| | |
|---|---|
| Oocytenpunktion mit anschließender IVM und IVF | 34 % |
| Embryoklonierung (durchschnittlich) | 25 % |
| Embryoklonierung mit primordialen Keimzellen | > 50 % |

| | |
|---|---|
| IVM = in vitro Maturation/Reifung IVF = in vitro Fertilisation | |

Die Erfindung wird nun unter Bezugnahme auf das lediglich zur Erläuterung gegebene Beispiel ausführlicher erläutert, das den Bereich der Erfindung nicht einschränken soll.

### Beispiel

### Isolierung primordialer Keimzellen beim Rinderfetus

Feten aus Uteri von geschlachteten Kalbinnen oder Kühen wurden freipräpariert und in PBS (Phosphate buffered Saline, ohne Ca²⁺/Mg²⁺, mit Penicillin/ Streptomycin, plus 10% fetales Kälberserum (FCS)) auf Eiswasser ins Labor gebracht. Die Feten wurden anschließend mehrmals mit frischem PBS gewaschen. Der Fetus wurde caudal der Vordergliedmaßen jeweils quergeteilt, es erfolgte eine mediane Öffnung der Bauchwand und Kranialverlagerung von Leber und Darmkonvolut. Anschließend wurden der nun freiliegende Mesonephros und die medial anliegenden Gonaden gewonnen und in frischem PBS gewaschen. Die Gonaden wurden mittels Pinzetten abpräpariert und erneut in PBS gewaschen. Dann wurden diese in 0,02%igem EDTA für 10 bis 20 Minuten inkubiert. Eine Inkubation in 0,4%iger Protease (Sigma, P 6911) für 3-8 Minuten bei 37-39°C erzielte die gleiche Wirkung.

Die Gonadenzellen wurden mittels einer Pipette in Kulturmedium mit zugesetzten Wachstumsfaktoren (Dulbecco's modifiziertes Eagle Medium (Gibco), ergänzt mit 15% FCS, 2mM L-Glutamin, 10⁻⁷ mMol β-Mercaptoethanol, 2 mMol nicht essentiellen Aminosäuren, LIF (Leukemia Inhibitory Factor, 1000 Units/ml), bFGF (basic Fibroblast Growth Factor, 10 ng/ml) und Penicillin/Streptomycin) umgesetzt wobei eine Zellkultur erhalten wurde. Alternativ wurden die Gonaden mehrmals mit einer 30 G-Nadel (Gauge) punktiert und die Zellen anschließend auf- und abpipettiert, bis eine Zellsuspension vorlag. Die Zellen wurden sodann in einer Tischzentrifuge bei 1100 U/min (160 g) 4 Minuten vorsichtig abzentrifugiert und anschließend erneut in Kulturmedium resuspendiert.

Die resuspendierten Zellen wurden sodann in 35 mm Petrischalen bei 37 bis 39°C, 5% CO₂ in wasserdampfgesättigter Luft bis zur Verwendung gezüchtet.

Zur Isolierung der Zellen für die Klonierung wurde die Zellsuspension in eine 4 cm Zellkulturschale überführt und für 20-22 Std. in Dulbecco's modifiziertem Eagle Medium (Gibco) (supra), bei 37-39°C, 5 % CO₂ gehalten. 15 Minuten vor Klonierungsbeginn wurden die Zellen kurz (1 - 2 Minuten) mit einer 0,1% igen Trypsinlösung (Sigma) behandelt, um sie vollständig in Suspension zu bringen.

Zur weiteren Selektion auf primordiale Keimzellen und deren Proliferation wurde die Kultur (enthaltend Keimzellen, Fibroblasten, Erythrozyten usw.) anschließend auf Feederzellen umgesetzt. Als Feederzellen konnten bovine Fibroblasten, nicht transfizierte STO-Zellen (ATCC CRL-1503) oder inaktivierte Gonadenanlagen erfolgreich eingesetzt werden. Das Kulturmedium war DMEM (Gibco, Nr. 074-2100A) (high Glucose), welches mit 15% FCS, 2 mMol nicht essentiellen Aminosäuren, 2 mMol L-Glutamin, 10⁻⁴ β-Mercaptoethanol, Penicillin/Streptomycin, 1000 IU/ml LIF und 10 ng/ml bFGF ergänzt worden war.

Alternativ wurden die primordialen Keimzellen aus der Zellkultur unter Verwendung einer Pipette selektiv in eine neue Schale umgesetzt, wodurch die Anzahl an "Fremd"-Zellen minimiert werden konnte.

Die primordialen Keimzellen, die als Kernspender verwendet werden sollen, lassen sich in der Zellsuspension als große (15-25µm), gelblich aussehende, irregulär oder rund geformte Zellen mit großem Kern identifizieren, die manchmal "Blebbing"-Phänomene zeigen. Diese Zellen konnten gezielt gewonnen werden.

### Additiver Gentransfer

In vitro rekombinierte Genkonstrukte, wie sie in der DE-OS-40 12 526 beschrieben sind, die hier unter Bezugnahme mit aufgenommen wird, werden durch konventionelle DNA-Mikroinjektion (Brem G., Transgenic Animals, Genetic Engineering of Animals, VCH Weinheim (1993), 83-170) in die Kerne isolierter primordialer Keimzellen oder durch bekannte Transformationsverfahren (Maniatis et al., Molecular Cloning, A Laboratory Manual, Cold Spring Harbor Laboratory, 1989) stabil integriert. Der Nachweis der Integration in den Zellen erfolgt mittels PCR und/oder Southern Blotting (Maniatis, vorstehend). Eine Expression in ausdifferenzierten Zellen zeigt, daß der Gentransfer erfolgreich gewesen ist.

### Klonierung

18-20 Std. nach dem Beginn der Reifung wurden aus dem Eierstock isolierte bovine Oozyten von den sie umgebenden Kumuluszellen befreit und innerhalb von zwei Stunden enukleiert (Molecular Reproduction and Development 42 (1995), 53-57). Ca. 20-22 Stunden nach Beginn der Reifung wurden wie vorstehend gewonnene primordiale Keimzellen mittels einer Transferpipette in den perivitellinen Raum von enukleierten Oozyten überführt und die sich dabei bildenden Karyoplast-Zytoplast-Komplexe (KZK) wurden jeweils für 10 µsek. einem doppelten elektrischen Puls von 2,1 kV/cm ausgesetzt, um die Fusion zu induzieren. Die KZKs wurden in Ham's F-12 Medium (Sigma) mit 10% FCS in einem Brutschrank gezüchtet. Die Fusion wurde 30 bis 60 Minuten nach dem Fusionspuls durch mikroskopische Untersuchung beurteilt.

24 Stunden nach Beginn der Reifung wurden die KZKs durch eine 5 minütige Inkubation in 7% Ethanol aktiviert und anschließend 5 Stunden in 10µg/ml Cycloheximid (Sigma C-7698) und 5 µg/ml Cytochalasin B (Sigma C-6762) gezüchtet. Anschließend wurden die KZKs in einem 100 µl Tropfen CR-1 Medium (Rosenkrans und First, 1991) mit 10% Östrus-Kuh-Serum umgesetzt. Die Tropfen wurden mit Paraffin-Öl überschichtet und für 7 bis 8 Tage bei 39°C in wasserdampfgesättigter Atmosphäre aus 5% CO₂, 5% O₂ und 90% N₂ gezüchtet.

**Tabelle : Primordiale Keimzellen (PKZ) als Kernspender**

| | KZK | Fusionierte KZK (%) | Teilung (%) | Blastozysten (%) |
|---|---|---|---|---|
| PKZ Fetus 50-57 Tage | 139 | 109 (74%) | 77 (71%) | 38 (35%) |
| PKZ Fetus 65-76 Tage | 143 | 128 (90%) | 81 (63%) | 32 (25%) |
| PKZ Fetus 95-105 Tage | 171 | 171 (87%) | 90 (60%) | 30(20%) |
| Embryonale Blastomeren | 111 | 108 (97%) | 66 (61%) | 3 (3%) |

Wie aus vorstehender Tabelle ersichtlich, war es bereits bei dem ersten Versuch möglich, eine Teilungsrate von bis zu 71 % und eine Blastozystenrate von bis zu 35 % zu erhalten.

### Embryotransfer

### Empfängermanagement

Als Empfänger wurden Kalbinnen verwendet, die folgende Kriterien erfüllten:
1. Aufzucht in IBR (bovines Herpesvirus Typ 1) unverdächtigen Betrieben;
2. serologische Untersuchung auf BHV-1-Antikörper (infektiöse bovine Rhinotracheitis /infektiöse pustulöse Vulvovaginitis) negativ;
3. serologische Untersuchung auf BVD (bovine Virus Diarrhoe)/MD-Antigen (Mucosal Disease) negativ;
4. dem Alter (13-16 Monate) entsprechende Körpermasseentwicklung;
5. eingetretene Geschlechtsreife; bei Tieren, die den Embryo austragen sollen, eingetretene Zuchtreife;
6. gynäkologische Untersuchung ohne pathologische Befunde;

Alle Empfänger erhielten unmittelbar nach Aufstallung Mineralstoffboli (All Trace, Ranching Consult GmbH), um die erfahrungsgemäß unzureichende Versorgung mit Selen, Kupfer und Cobalt auszugleichen (Wittkowski et al., Zur Selensupplementierung bei Färsen; Jahrestagung der Arbeitsgemeinschaft Embryotransfer Deutschland (AET-d). 13.06.-14.06.1996, Marktredwitz).

BVD-Antikörper negative Tiere wurden gegen BVD/MD immunisiert (Rumilis^{®}, Intervet), um das Infektionsrisiko bei Übertragung von Embryonen (Mödl et al., Control of bovine viral diarrhea virus in abattoir ovaries for in vitro fertilization (IVF) or cloning programs. 11e Reunion A.E.T.E.-Hannover, 8-9 September 1995) bzw. nach Verbringen in Stallungen mit unbekanntem BVD-Status zu minimieren. Die Fütterung erfolgte ad libitum mit Grassilage, Heu und Stroh. Entwurmungen wurden im Frühjahr und Herbst mit Ivermectin (Ivomec^{®}, MSD Agvet) durchgeführt. Die Unterbringung der Empfänger erfolgt zum Teil in Laufstall- (Offenstall, Gruppengröße 6 Tiere) und zum Teil in Anbindehaltung.

### Empfängervorbereitung

Die Übertragung der in vitro hergestellten Embryonen erfolgte auf Zyklus-synchrone Empfänger, d.h. das Stadium des Sexualzyklus entspricht dem Alter des zu übertragenden Embryos. Dabei wird der Tag der Brunst als Zyklustag 0 bezeichnet. Die Brunstsynchronisation erfolgte im Diöstrus durch die einmalige intramuskuläre Applikation eines Prostaglandin F₂-α-Analogons (2,0 ml Estrumate®, Mallinckrodt Veterinary). Kalbinnen, bei denen mittels rektaler Palpation kein funktionelles Corpus luteum diagnostizierbar war, wurden nicht für die Brunstsynchronisation verwendet. Die Brunst tritt erfahrungsgemäß 2-3 Tage post applicationem ein und wird anhand des Brunstverhaltens und des Scheidenbefundes beurteilt.

### Embryotransfer

Die in vitro produzierten Embryonen wurden nach 7-tägiger Kultur auf geeignete Empfänger transferiert. Dazu wurden die Embryonen identifiziert, qualitativ beurteilt, Zona geschlitzt, in ein geeignetes Transfermedium umgesetzt und anschließend in Minipailletten (Minitüb) aufgezogen. Als Transfermedien wurden PBS + 10% fetales Kälberserum (FCS, Biochrom), Ovum Culture Medium (ICP, Neuseeland) + 10% FCS oder TL-Hepes + 10% FCS verwendet.

Die verschlossenen Pailletten wurden bis zum Transfer, der innerhalb von ca. 90 Minuten stattfinden sollte, bei 37,8°C in einem Miniinkubator gelagert.

Die Eignung der Empfänger wurde anhand folgender Kriterien beurteilt:

Die Tiere wurden etwa 7 Tage vor dem Transfer in Brunst beobachtet, wobei die Asynchronität 24 Std. nicht überschreiten soll (Hasler et al., Theriogenology 43 (1995), 141-152). Das Vorhandensein und die Größe eines funktionellen Gelbkörpers wurde entsprechend bewertet (Assey et al., Theriogenology 39 (1993), 1321-1330).

Die verwendeten Tiere wiesen keine Anzeichen einer Erkrankung des Genitaltraktes auf.

Nach der Auswahl wurde eine Epiduralanästhesie (2,0 ml Lidocain^{®}, Albrecht) vorgenommen und das äußere Genitale sorgfältig mit trockenem Papier gereinigt. Anschließend wurde der körperwarme Transferkatheter (Minitüb) mit einer Paillette beschickt und mit einer Plastikschutzhülle (Sanisheath, Minitüb) versehen. Der Transfer erfolgte unblutig unter rektaler Kontrolle der Cervixpassage und der Katheterposition in die Spitze des ipsilateralen Uterushornes (Reichenbach et al., J. Reprod. Fertil. 95 (1992), 363-370). Dabei wurde die Plastikschutzhülle erst am äußeren Muttermund mit dem Transferkatheter perforiert, um eine Keimverschleppung aus der Vagina in den Uterus zu vermeiden. War der Transfer mehrerer Embryonen auf einen Empfänger vorgesehen, so wurden diese bilateral abgesetzt. Dazu wurde der Transferkatheter bis in das Corpus uteri zurückgezogen, der Mandrin mit der entleerten Paillette entfernt, eine neue Paillette mit Embryo(nen) in den Katheter geschoben und im contralateralen Uterushorn positioniert.

Unmittelbar nach dem Transfer wurden alle relevanten Daten (Lebensnummer des Empfängers, Herkunft, Anzahl und Qualität der Embryonen usw.) dokumentiert.

### Trächtigkeitsuntersuchung

21 Tage nach der Brunst, also 14 Tage nach dem Embryotransfer, wurde eine Brunstkontrolle vorgenommen und der Progesterongehalt im Blutserum festgestellt. Werte unter 0,1 ng/ml werden als sicher nicht trächtig angesehen. Bei Progesteronwerten über 2,0 ng/ml kann mit einer Trächtigkeit gerechnet werden. Die erste direkte Trächtigkeitsuntersuchung wurde um den 35. Tag mittels Ultraschall und die zweite manuell um den 42. Trächtigkeitstag vorgenommen.

## Patentansprüche

1. Verfahren zur Herstellung eines tierischen Embryos, das die folgenden Schritte umfasst:
(a) Gewinnen einer primordialen Keimzelle, wobei die primordiale Keimzelle ein Alter von 50 bis 105 Tagen aufweist;
(b) Vereinen des Kerns der primordialen Keimzelle mit einer geeigneten Empfängerzelle;
(c) Aktivieren der in Schritt (b) erhaltenen Zelle;
(d) Züchten der so erhaltenen Zelle für einen Zeitraum, damit sich eine Blastocyste bildet; und
(e) Aufschlitzen der Zona pellucida;
wobei der tierische Embryo ein Ungulat ist.

2. Verfahren nach Anspruch 1, bei dem die geeignete Empfängerzelle eine enukleierte Eizelle ist.

3. Verfahren nach einem der vorhergehenden Ansprüche, bei dem die primordiale Keimzelle mit der geeigneten Empfängerzelle fusioniert wird.

4. Verfahren nach Anspruch 3, bei dem das Tier ein Rind ist.

5. Verfahren nach einem der vorhergehenden Ansprüche, bei dem die primordiale Keimzelle transgen ist.

6. Verfahren nach Anspruch 5, bei dem die primordiale Keimzelle ein pharmazeutisch oder ernährungsphysiologisch interessantes Gen enthält.

7. Verfahren nach Anspruch 5 oder 6, bei dem das Genprodukt des eingebrachten Gens in die Milch sezerniert wird.

8. Verfahren nach Anspruch 7, bei dem das Transgen Chymosin oder Trypsin ist.

9. Verfahren nach einem der Ansprüche 5 bis 8, bei dem das Transgen unter der Kontrolle eines endogenen Promotors liegt.

10. Verfahren nach einem der Ansprüche 5 bis 8, bei dem das Transgen oder ein endogenes Gen unter der Kontrolle eines exogenen Fremdpromotors oder eines Spezies endogenen Promotors liegt.

## Claims

1. Method for the preparation of an animal embryo, comprising the following steps:
(a) Collecting a primordial germ cell, wherein the primordial germ cell is 50 to 105 days old;
(b) Combining the nucleus of the primordial germ cell with a suitable recipient cell;
(c) Activating of the cell obtained in step (b);
(d) Growing of the so obtained cell for a period of time for forming a blastocyst;
(e) Cutting the zona pellucida,
wherein the animal embryo is an ungulate.

2. Method according to claim 1, wherein the suitable recipient cell is an enucleated egg cell.

3. Method according to any of the preceding claims, wherein the primordial germ cell is fused with the suitable recipient cell.

4. Method according to claim 3, wherein the animal is cattle.

5. Method according to any of the preceding claims, wherein the primordial germ cell is transgenic.

6. Method according to claim 5, wherein the primordial germ cell contains a pharmaceutically or nutritional physiologically interesting gene.

7. Method according to claim 5 or 6, wherein the gene product of the introduced gene is secreted into the milk.

8. Method according to claim 7, wherein the transgene is Chymosin or Trypsin.

9. Method according to any of the claims 5 to 8, wherein the transgene is under the control of an endogenous promoter.

10. Method according to any of the claims 5 to 8, wherein the transgene or an endogenous gene is under the control of an exogenous foreign promoter or of a species endogenous promoter.

## Revendications

1. Procédé de préparation d'un embryon animal qui comporte les étapes suivantes :
(a) acquérir une cellule germinale primordiale, laquelle cellule germinale primordiale présente un âge de 50 à 105 jours ;
(b) unir le noyau de la cellule germinale primordiale avec cellule d'accueil adéquate ;
(c) activer la cellule obtenue dans l'étape (b) ;
(d) élever la cellule ainsi obtenue pour une période permettant qu'un blastocyte se forme ; et
(e) ouvrir la « zona pellucida », dans lequel l'embryon animal est un ongulé.

2. Procédé selon la revendication 1 dans lequel la cellule d'accueil adéquate est un oocyte énucléé.

3. Procédé selon l'une des revendications précédentes dans lequel la cellule germinale primordiale est fusionnée avec la cellule d'accueil adéquate.

4. Procédé selon la revendication 3 dans lequel animal est un bovidé.

5. Procédé selon l'une des revendications précédentes dans lequel la cellule germinale primordiale est transgénique.

6. Procédé selon la revendication 5 dans lequel la cellule germinale primordiale contient un gène intéressant sur le plan pharmaceutique ou physiologique nutritionnel.

7. Procédé selon la revendication 5 ou 6 dans lequel le produit génique du gène introduit est sécrété dans le lait.

8. Procédé selon la revendication 7 dans lequel le transgène est la chymosine ou la trypsine.

9. Procédé selon l'une des revendications 5 à 8 dans lequel le transgène est sous le contrôle d'un promoteur endogène.

10. Procédé selon l'une des revendications 5 à 8 dans lequel le transgène ou un gène endogène est sous le contrôle d'un promoteur exogène étranger ou d'un promoteur endogène d'espèce.
